# FASCICULE DE BREVET EUROPEEN

(11) **EP 0 706 044 B1**
(45) Date de publication et mention de la délivrance du brevet: **01.12.1999**
(21) Numéro de dépôt: 95420229.7
(22) Date de dépôt: 08.08.1995
(51) Int. Cl.: G01N 21/85, A61M 1/36

(54) **Dispositif de détection d'un conduit et de détermination d'au moins une caractéristique de son contenu**
Vorrichtung zum Nachweis eines Rohres und zur Bestimmung mindestens einer Charakteristik dessen Inhalts
Device for detection of a conduit and determination of at least one characteristic of its contents

(30) Priorité: 07.10.1994 FR 9412252
(43) Date de publication de la demande: 10.04.1996
(73) Titulaire: HOSPAL INDUSTRIE, F-69883 Meyzieu Cédex (FR)
(72) Inventeur: Chevallet, Jacques, F-69360 Serezin Du Rhone (FR)
(74) Mandataire: Lejeune, Daniel

(56) Documents cités:
- DE-A- 3 910 250
- FR-A- 2 660 755
- US-A- 4 857 050
- US-A- 5 261 874

## Description

La présente invention a pour objet un dispositif de détection d'un conduit et de détermination d'au moins une caractéristique de son contenu.

Dans tous les appareils de traitement du sang par circulation extracorporelle où le sang d'un patient est mis en circulation dans un échangeur à membrane semi-perméable (dialyseur, filtre pour plasmaphérèse ou hémofiltration, par exemple), il est important de pouvoir vérifier en cours de traitement, de façon continue, que la membrane de l'échangeur ne subit pas de rupture, notamment sous l'effet de la pression transmembranaire créée dans l'échangeur pour provoquer le passage de liquide plasmatique au travers de la membrane, du compartiment sang de l'échangeur vers le compartiment de liquide usé.

Un moyen couramment utilisé pour vérifier l'intégrité de la membrane des échangeurs utilisés aux fins qui viennent d'être mentionnées consiste à placer, sur une canalisation de sortie du compartiment de liquide usé de l'échangeur, un dispositif de détection de sang dans le liquide circulant dans la canalisation. Généralement, ces dispositifs de détection sont également prévus pour détecter la présence de la canalisation, quel que soit son contenu (air ou solution saline), à titre de condition de fonctionnement sine qua non.

Le brevet allemand 3 910 250 décrit un détecteur de ce type comprenant :
- un siège pour loger une portion de canalisation, comprenant une première et une seconde zones opposées par rapport à l'axe longitudinal de la canalisation lorsque celle-ci est logée dans le siège ;
- des moyens d'émission de lumière et des moyens de réception sensibles à la lumière émise par les moyens d'émission, les moyens d'émission et les moyens de réception étant disposés dans la première zone pour avoir des directions d'émission et de réception parallèles ; et
- des moyens de déviation de lumière disposés dans la deuxième zone pour renvoyer vers les moyens de réception au moins une partie de la lumière émise par les moyens d'émission.

Dans les modes de réalisation décrits, les moyens de déviation sont constitués par deux miroirs disposés en vis-à-vis et formant un angle de 45° par rapport à la direction d'émission et la direction de réception, respectivement. La lumière émise par les moyens d'émission est réfléchie deux fois avant d'atteindre les moyens de réception, et le trajet optique entre les moyens d'émission et de réception est toujours le même, qu'une canalisation ait été engagée dans le siège ou non, et quelle que soit la nature du fluide présent dans la canalisation (gaz ou liquide). Avec ce dispositif, une différence de nature ou de qualité du fluide présent dans la canalisation (liquide ou gaz, liquide contenant ou non du sang) se traduit, dans la limite de la sensibilité des moyens de réception et du circuit de traitement de signal qui lui est associé, par une atténuation différente de la lumière émise par les moyens d'émission au niveau des moyens de réflexion.

Un but de l'invention est de réaliser un dispositif de détection du type mentionné ci-dessus, qui ait une capacité accrue de discrimination entre des fluides de différentes natures ou de différente qualité, et qui soit en outre adapté à détecter des quantités de sang très faibles dans un liquide circulant dans une canalisation de diamètre très réduit.

Pour atteindre ce but, on prévoit, selon l'invention un dispositif de détection d'un conduit et de détermination d'au moins une caractéristique de son contenu comprenant :
- un siège pour loger au moins une portion du conduit, comprenant une première et une seconde zones opposées par rapport à un évidement destiné à recevoir le conduit ;
- des moyens d'émission de lumière, disposés dans la première zone, ayant une direction préférentielle d'émission orientée vers l'évidement et des moyens de réception sensibles à la lumière émise par les moyens d'émission, ayant une direction préférentielle de réception orientée vers l'évidement ; et
- des premiers moyens de déviation de lumière pour dévier, en direction de la première zone, au moins une partie de la lumière émise par les moyens d'émission ;
caractérisé en ce qu'il comporte des seconds moyens de déviation de lumière pour dévier en direction de la deuxième zone au moins une partie de la lumière déviée par les premiers moyens de déviation de façon que, lorsqu'un conduit contenant un liquide sensiblement transparent est engagé dans le siège, au moins une partie de la lumière émise par les moyens d'émission atteigne les moyens de réception après avoir traversé le conduit au moins deux fois en oblique part rapport à un axe longitudinal de l'évidement.

Ce dispositif présente un double avantage lié aux déviations multiples imposant à au moins une partie de la lumière émise par les moyens d'émission de traverser plusieurs fois le conduit en oblique. D'une part, grâce à l'allongement du trajet optique à l'intérieur du conduit, il est possible de détecter des concentrations de sang dans un liquide usé beaucoup plus faibles qu'avec les dispositifs classiques, ou dans des conduits de section beaucoup plus faibles. D'autre part, l'obliquité, par rapport à plusieurs milieux d'indice de réfraction différents disposés en couches parallèles, de portions de trajets optiques multiples, permet d'agencer les moyens d'émission, les moyens de réception et les premiers et les seconds moyens de déviation les uns par rapport aux autres de façon que la discrimination entre les différentes situations de détection (présence ou absence de conduit dans le siège, nature du fluide dans le conduit) puisse se faire de façon plus tranchée. Ainsi, dans un mode de réalisation de l'invention, les moyens d'émission, les moyens de réception et les premiers et les seconds moyens de dérivation sont agencés de façon que :
- en l'absence de conduit dans le siège, au moins une partie de la lumière émise par les moyens d'émission atteigne les moyens de réception après simple déviation ;
- lorsqu'un conduit contenant un gaz est engagé dans le siège, les moyens de réception ne reçoivent sensiblement aucune lumière provenant des moyens d'émission ; et
- lorsqu'un conduit contenant un liquide sensiblement transparent est engagé dans le siège, au moins une partie de la lumière émise par les moyens d'émission atteigne les moyens de réception après avoir traversé quatre fois le conduit.

Selon une caractéristique de l'invention, le dispositif est utilisé comme détecteur de sang dans une canalisation de liquide usé d'un appareil de traitement du sang par circulation extracorporelle, et la longueur d'onde de la lumière émise par les moyens d'émission est choisie pour être hors du spectre de la lumière visible et ne pas être absorbée par un liquide ayant la coloration d'un ultrafiltrat sanguin éventuellement dilué dans un solution saline.

Grâce à cette disposition, l'atténuation de la lumière émise par les moyens d'émission, au niveau des moyens de réception, ne peut résulter que de sa diffraction sur les globules rouges.

D'autre caractéristiques et avantages de l'invention apparaîtront à la lecture de la description qui va suivre. On se reportera aux dessins annexés sur lesquels :
La figure 1 est une vue en coupe d'un premier mode de réalisation d'un dispositif de détection selon l'invention ;
Les figures 2, 3, 4 illustrent le fonctionnement du dispositif de la figure 1, dans trois situations de détection différente : en l'absence de conduit, en présence d'un conduit rempli de gaz et en présence d'un conduit rempli de liquide ;
La figure 5 est une vue en coupe d'un deuxième mode de réalisation de l'invention ; et
La figure 6 est une vue en coupe d'un troisième mode de réalisation de l'invention.

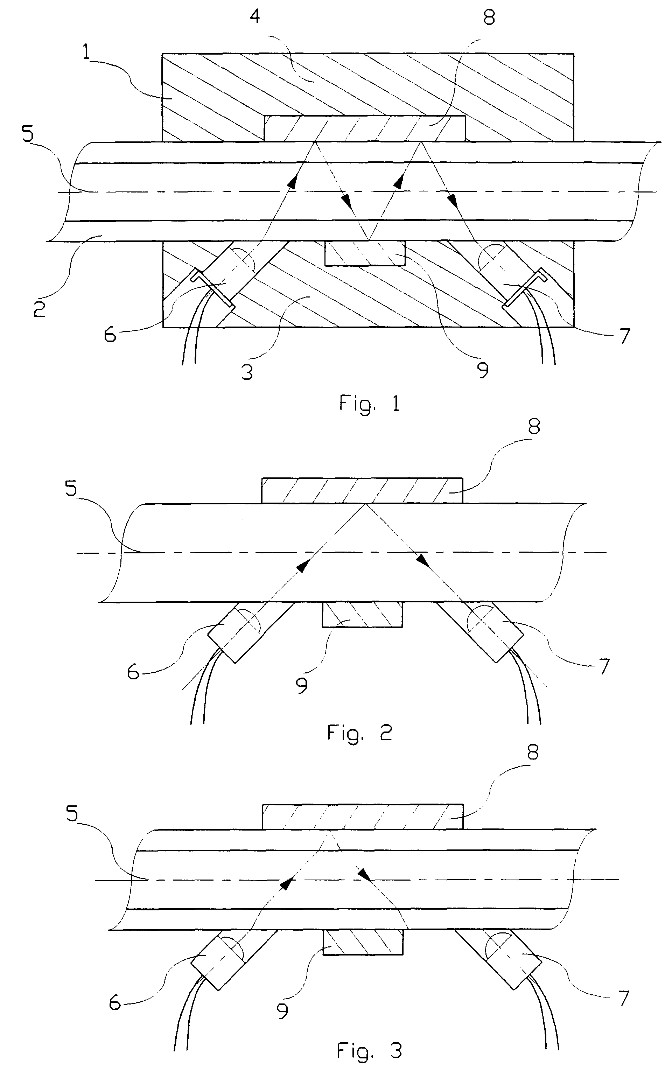

Le dispositif de détection représenté sur la figure 1 comprend un siège 1 constitué par un bloc de matière plastique comprenant un évidement allongé de section transversale rectangulaire configuré et dimensionné pour loger et retenir un conduit transparent 2, qui peut être, par exemple, une portion d'une canalisation en tube de PVC. Le siège 1 comprend deux zones 3, 4 diamétralement opposées par rapport à un axe longitudinal 5 de l'évidement avec lequel coïncide sensiblement un axe longitudinal de la canalisation 2 lorsque la canalisation 2 est engagée dans l'évidement. Dans une première zone 3 sont disposés des moyens d'émission de lumière 6, tels qu'une diode électroluminescente, ayant une direction d'émission préférentielle coupant l'axe longitudinal 5 de l'évidement à 45°, et des moyens de réception de lumière 7, tels qu'un phototransistor, ayant une direction de réception préférentielle coupant l'axe longitudinal 5 de l'évidement à 45°. La diode 6 et le transistor 7 sont connectés à un circuit de traitement de signal (non représenté), prévu pour calculer l'atténuation de la lumière reçue par le transistor et pour comparer l'atténuation calculée à des plages de valeurs de seuil admissibles. Dans la deuxième zone 4, sont disposés des premiers moyens de déviation de lumière 8, tels qu'un miroir plan, pour dévier en direction de la première zone 3, au moins une partie de la lumière émise par la diode 6.

Conformément à l'invention, de seconds moyens de déviation de lumière 9, tels qu'un second miroir plan, sont disposés dans la première zone 3, pour dévier, en direction de la deuxième zone 4, au moins une partie de la lumière réfléchie par le premier miroir 8. Les surfaces réfléchissantes du premier et du second miroirs 8, 9 sont parallèles entre elles ainsi qu'à l'axe longitudinal 5 de l'évidement.

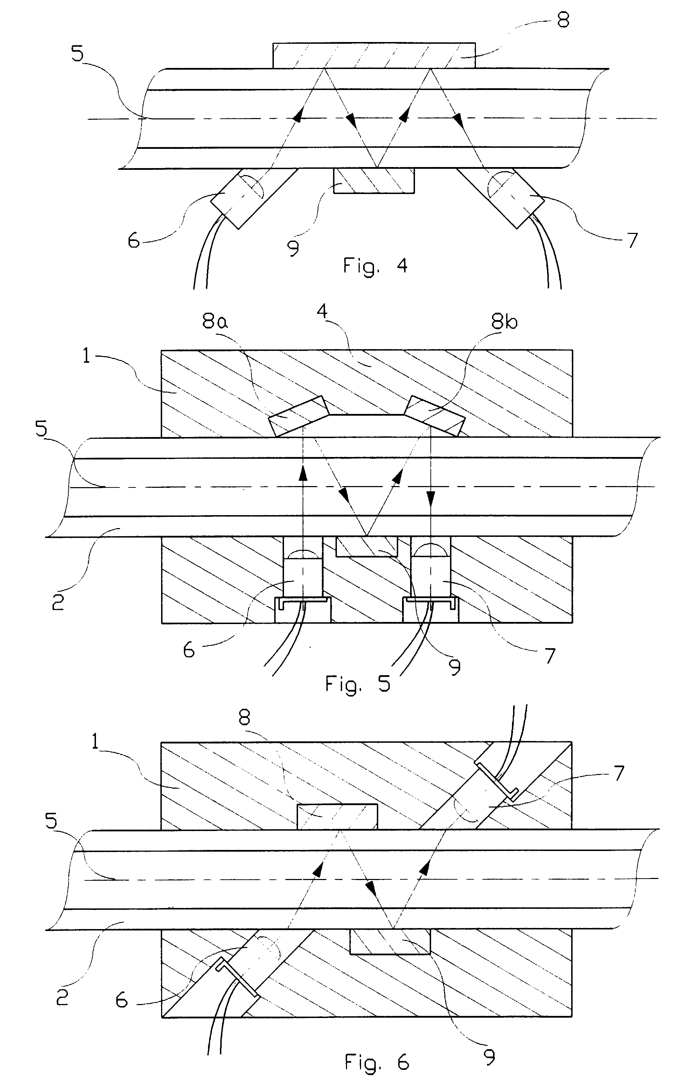

Conformément à l'invention, la distance entre la diode 6 et le phototransistor 7, de même que le positionnement des miroirs 8, 9 l'un par rapport à l'autre et par rapport à la diode 6 et au phototransistor 7, sont choisis en tenant compte du diamètre de la canalisation 2, de l'épaisseur de ses parois et de l'indice de réfraction des différents milieux optiques traversés par la lumière pour que :
- en l'absence de canalisation dans le siège 1, la lumière émise par la diode 6 soit déviée, par simple réflexion sur le premier miroir 8, en direction du phototransistor (voir figure 2). Un des intérêts de cette disposition est qu'elle permet d'étalonner le détecteur avant toute utilisation et de tenir compte du vieillissement de la diode 6 dans les comparaisons et calculs effectués par le circuit de traitement de signal auquel la diode 6 et le transistor 7 sont connectés ;
- lorsqu'une canalisation 2 remplie d'air est engagée dans le siège 1, la lumière émise par la diode 6 ne parvient pas au phototransistor 7 (voir figure 3);
- lorsqu'une canalisation 3 remplie de liquide est engagée dans le siège 1, au moins une partie de la lumière émise par la diode 6 est déviée par triple réflexion en direction du phototransistor 7, d'abord sur le premier miroir 8, puis sur le second miroir 9, enfin sur le premier miroir 8 (voir figures 1 et 4).

Sur les figures 1 à 4 on a pris l'exemple d'un tube de PVC transparent (indice : 1,49) où est mise en circulation une solution saline (indice : 1,33).

On remarque que, avec ce dispositif, à chaque situation de détection significative (présence ou absence de canalisation dans le siège 1, gaz ou liquide dans la canalisation), correspond un trajet optique différent de la lumière émise par la diode 6. Cette caractéristique aide à discriminer les situations de détection.

Par ailleurs, l'allongement du trajet optique dans le cas où la canalisation est remplie de liquide permet de détecter de très faibles concentrations de sang et, par ailleurs, d'effectuer une telle détection dans des canalisations de diamètres très réduits. A titre d'exemple, le dispositif de détection représenté sur la figure 1, permet de détecter, dans une canalisation de PVC de 2,8 millimètres de diamètre intérieur et de 4,6 millimètres de diamètre extérieur où circule, à un débit de 58 millitres/minute, un liquide de dialyse mélangé à de l'ultrafiltrat, une fuite de sang (hématocrite : 35 %) ayant un débit de 0,1 millitre/minute, soit une dilution de 1,7 pour mille.

Dans l'utilisation qui vient d'être mentionnée, pour conférer au dispositif de détection selon l'invention une sélectivité et une précision accrue, on choisit la longueur d'onde de la lumière émise par la diode hors du spectre du visible (pour éviter les interférences avec la lumière ambiante) et hors du spectre des longueurs d'ondes susceptibles d'être absorbées par un liquide ayant la couleur de l'ultrafiltrat sanguin. A titre d'exemple, une lumière infrarouge ayant une longueur d'onde d'environ 890 nanomètres remplit ces conditions.

La figure 5 représente un second mode de réalisation de l'invention qui se différencie de celui qui vient d'être décrit en ce que la direction d'émission et la direction de réception préférentielles de la diode 6 et du phototransistor 7 sont parallèles entre elles et perpendiculaires à l'axe longitudinal 5 de l'évidement, et en ce que les premiers moyens de déviation de lumière comportent deux miroirs plans 8a et 8b dont les surfaces réfléchissantes sont inclinées par rapport à l'axe longitudinal 5 de l'évidement de façon à pouvoir réfléchir respectivement au moins une partie de la lumière émise par la diode 6, et au moins une partie de la lumière réfléchie par le second miroir 9.

Avec ce dispositif, en l'absence de canalisation dans le siège 1, au moins une partie de la lumière émise par la diode 6 est déviée, par simple réflexion sur le miroir 8a, vers le phototransistor 7. Par ailleurs, lorsqu'une canalisation 2 remplie de gaz est engagée dans le siège 1, aucune lumière n'est déviée par les miroirs en direction du phototransistor 7. Le trajet optique représenté, qui coupe quatre fois l'axe longitudinal 5 du conduit 2, correspond au cas où une canalisation 2 remplie de liquide est engagée dans le siège 1.

Dans le dispositif de détection représenté sur la figure 6, la diode 6 et le phototransistor 7 sont respectivement disposés dans la première zone 3 et dans la deuxième zone 4 de façon que la direction préférentielle d'émission de l'une et la direction préférentielle de réception de l'autre coupent l'axe longitudinal 5 de l'évidement à 45°. Comme dans le dispositif de la figure 1, le premier et le second miroirs 8, 9 sont parallèles entre eux et à l'axe longitudinal 5 de l'évidement. La diode 6, le phototransistor 7 et les miroirs 8, 9 sont agencés les uns par rapport aux autres de façon que, lorsqu'une canalisation 2 remplie de liquide est engagée dans le siège 1, au moins une partie de la lumière émise par la diode 6 atteigne le phototransistor 7 après avoir traversé trois fois la canalisation 2 en oblique (trajet optique représenté sur la figure). En l'absence de canalisation dans le siège 1, et lorsqu'une canalisation 2 remplie de gaz est engagée dans le siège 1, aucune lumière n'est déviée par les miroirs 8, 9 en direction du phototransistor 7

L'invention n'est pas limitée aux modes de réalisation représentés et elle est susceptible de variantes. En particulier, les moyens de dérivation de lumière, au lieu des miroirs décrits, pourraient être constitués par des prismes.

Par ailleurs, bien que l'invention ait été décrite dans son application aux appareils de traitement de sang par circulation extracorporelle, il va de soi qu'elle peut être utilisée dans tous les domaines techniques où se posent des problèmes similaires.

## Revendications

1. Dispositif de détection d'un conduit (2) et de détermination d'au moins une caractéristique de son contenu comprenant :
- un siège (1) pour loger au moins une portion du conduit (2), comprenant une première et une seconde zones opposées (3, 4) par rapport à un évidement destiné à recevoir le conduit (2) ;
- des moyens d'émission de lumière (6), disposés dans la première zone (3), ayant une direction préférentielle d'émission orientée vers l'évidement et des moyens de réception (7) sensibles à la lumière émise par les moyens d'émission (6), ayant une direction préférentielle de réception orientée vers l'évidement ; et
- des premiers moyens de déviation de lumière (8) pour dévier, en direction de la première zone (3), au moins une partie de la lumière émise par les moyens d'émission (6) ;
caractérisé en ce qu'il comporte des seconds moyens de déviation de lumière (9) pour dévier en direction de la deuxième zone (4) au moins une partie de la lumière déviée par les premiers moyens de déviation (8) de façon que, lorsqu'un conduit (2) contenant un liquide sensiblement transparent est engagé dans le siège (1), au moins une partie de la lumière émise par les moyens d'émission (6) atteigne les moyens de réception (7) après avoir traversé le conduit (2) au moins deux fois en oblique part rapport à un axe longitudinal (5) de l'évidement.

2. Dispositif selon la revendication 1, caractérisé en ce que les moyens de réception (7) sont disposés dans la première zone (3) et en ce que les moyens d'émission (6), les moyens de réception (7), les premiers moyens de déviation (8) et les seconds moyens de déviation (9) sont agencés les uns par rapport aux autres de façon que, lorsqu'un conduit (2) contenant un liquide sensiblement transparent est engagé dans le siège (1), au moins une partie de la lumière émise par les moyens d'émission (6) atteigne les moyens de réception (7) après avoir traversé quatre fois le conduit (2).

3. Dispositif selon une des revendications 1 et 2, caractérisé en ce que les moyens d'émission (6), les moyens de réception (7), les premiers moyens de déviation (8) et les seconds moyens de déviation (9) sont agencés les uns par rapport aux autres de façon que, en l'absence de conduit (2) dans le siège (1), au moins une partie de la lumière émise par les moyens d'émission (6) atteigne les moyens de réception (7) après simple déviation.

4. Dispositif selon une des revendications 1 à 3, caractérisé en ce que les moyens d'émission (6), les moyens de réception (7), les premiers moyens de déviation (8) et les seconds moyens de déviation (9) sont agencés les uns par rapport aux autres de façon que, lorsqu'un conduit (2) contenant un gaz est engagé dans le siège (1), les moyens de réception (7) ne reçoivent aucune lumière provenant des moyens d'émission (6).

5. Dispositif selon une des revendications 2 à 4, caractérisé en ce que :
- les premiers moyens de réflexion et les seconds moyens de réflexion sont des miroirs plans (8, 9) ayant leurs surfaces réfléchissantes disposées dans des plans parallèles à l'axe longitudinal (5) de l'évidement ;
- les moyens d'émission (6) sont disposés pour avoir une direction préférentielle d'émission formant un angle de environ 135°, respectivement 45°, par rapport à l'axe longitudinal (5) de l'évidement, et
- les moyens de réception (7) sont disposés pour avoir une direction préférentielle de réception formant un angle de environ 45°, respectivement 135°, par rapport à l'axe longitudinal (5) de l'évidement.

6. Dispositif selon la revendication 1, caractérisé en ce que les moyens de réception (7) sont disposés dans la deuxième zone (4) et en ce que les moyens d'émission (6), les moyens de réception (7), les premiers moyens de déviation (8) et les seconds moyens de déviation (9) sont agencés les uns par rapport aux autres de façon que, lorsqu'un conduit (2) contenant un liquide sensiblement transparent est engagé dans le siège (1), au moins une partie de la lumière émise par les moyens d'émission (6) atteigne les moyens de réception (7) après avoir traversé trois fois le conduit (2).

7. Dispositif selon une des revendications 1 à 6 pour une utilisation comme détecteur de sang dans une canalisation de liquide usé d'un appareil de traitement du sang par circulation extracorporelle, caractérisé en ce que la longueur d'onde de la lumière émise par les moyens d'émission (6) est choisie pour être hors du spectre de la lumière visible et ne pas être absorbée par un liquide ayant la coloration d'un ultrafiltrat sanguin éventuellement dilué dans un solution saline.

## Patentansprüche

1. Vorrichtung zur Erfassung einer Leitung (2) und zur Bestimmung von mindestens einer Eigenschaft ihres Inhalts, die folgendes umfaßt:
- eine Aufnahme (1) zum Unterbringen mindestens eines Teils der Leitung (2), die eine erste und eine zweite Zone (3, 4) aufweist, die sich bezüglich einer zur Aufnahme der Leitung (2) bestimmten Ausnehmung gegenüberliegen;
- in der ersten Zone (3) angeordnete Lichtabstrahlmittel (6) mit einer Vorzugsabstrahlrichtung, die zur Ausnehmung ausgerichtet ist, und Empfangsmittel (7), die für das von den Abstrahlmitteln (6) abgestrahlte Licht empfindlich sind und eine zur Ausnehmung ausgerichtete Vorzugsempfangsrichtung haben; und
- erste Lichtumlenkungsmittel (8) zum Umlenken mindestens eines Teils des von den Abstrahlmitteln (6) abgestrahlten Lichts in Richtung der ersten Zone (3);
dadurch gekennzeichnet, daß sie zweite Lichtumlenkungsmittel (9) zur Umlenkung mindestens eines Teils des durch die ersten Umlenkungsmittel (8) umgelenkten Lichts in Richtung der zweiten Zone (4) enthält, so daß, wenn eine eine im wesentlichen durchsichtige Flüssigkeit enthaltende Leitung (2) in der Aufnahme (1) in Eingriff steht, mindestens ein Teil des von den AbstrahlmitteIn (6) abgestrahlten Lichts die Empfangsmittel (7) erreicht, nachdem er die Leitung (2) mindestens zweimal schräg zu einer Längsachse (5) der Ausnehmung durchquert hat.

2. Vorrichtung nach Anspruch 1, dadurch gekennzeichnet, daß die Empfangsmittel (7) in der ersten Zone (3) angeordnet sind und daß die Abstrahlmittel (6), die Empfangsmittel (7), die ersten Umlenkungsmittel (8) und die zweiten Umlenkungsmittel (9) bezüglich einander so angeordnet sind, daß, wenn eine eine im wesentlichen durchsichtige Flüssigkeit enthaltende Leitung (2) in der Aufnahme (1) in Eingriff steht, mindestens ein Teil des von den AbstrahlmitteIn (6) abgestrahlten Lichts die Empfangsmittel (7) nach vierfacher Durchquerung der Leitung (2) erreicht.

3. Vorrichtung nach Anspruch 1 oder 2, dadurch gekennzeichnet, daß die Abstrahlmittel (6), die Empfangsmittel (7), die ersten Umlenkungsmittel (8) und die zweiten Umlenkungsmittel (9) bezüglich einander so angeordnet sind, daß bei Abwesenheit einer Leitung (2) in der Aufnahme (1) mindestens ein Teil des von den Abstrahlmitteln (6) abgestrahlten Lichts die Empfangsmittel (7) nach einfacher Durchquerung der Leitung erreicht.

4. Vorrichtung nach einem der Ansprüche 1 bis 3, dadurch gekennzeichnet, daß die Abstrahlmittel (6), die Empfangsmittel (7), die ersten Umlenkungsmittel (8) und die zweiten Umlenkungsmittel (9) bezüglich einander so angeordnet sind, daß, wenn eine Gas enthaltende Leitung (2) in der Aufnahme (1) in Eingriff steht, die Empfangsmittel (7) kein von den Abstrahlmitteln (6) stammendes Licht empfangen.

5. Vorrichtung nach einem der Ansprüche 2 bis 4, dadurch gekennzeichnet, daß
- es sich bei den ersten Reflexionsmitteln und den zweiten Reflexionsmitteln um Planspiegel (8, 9) handelt, deren Reflexionsflächen in parallel zu der Längsachse (5) der Ausnehmung verlaufenden Ebenen angeordnet sind;
- die Abstrahlmittel (6) so angeordnet sind, daß ihre Vorzugsabstrahlrichtung mit der Längsachse (5) der Ausnehmung einen Winkel von ca. 135° bzw. 45° bildet, und
- die Empfangsmittel (7) so angeordnet sind, daß ihre Vorzugsempfangsrichtung mit der Längsachse (5) der Ausnehmung einen Winkel von ca. 45° bzw. 135° bildet.

6. Vorrichtung nach Anspruch 1, dadurch gekennzeichnet, daß die Empfangsmittel (7) in der zweiten Zone (4) angeordnet sind und die Abstrahlmittel (6), die Empfangsmittel (7), die ersten Umlenkungsmittel (8) und die zweiten Umlenkungsmittel (9) bezüglich einander so angeordnet sind, daß, wenn eine eine im wesentlichen durchsichtige Flüssigkeit enthaltende Leitung (2) in der Aufnahme (1) in Eingriff steht, mindestens ein Teil des von den Abstrahlmitteln (6) abgestrahlten Lichts die Empfangsmittel (7) nach dreifacher Durchquerung der Leitung (2) erreicht.

7. Vorrichtung nach einem der Ansprüche 1 bis 6 zur Verwendung als Erfassungsvorrichtung von Blut in einer Leitung für verbrauchte Flüssigkeit eines Geräts zur Behandlung von Blut durch extrakorporale Zirkulation, dadurch gekennzeichnet, daß die Wellenlänge des von den Abstrahlmitteln (6) abgestrahlten Lichts so ausgewählt ist, daß sie sich außerhalb des Spektrums des sichtbaren Lichts befindet und nicht von einer Flüssigkeit mit der Farbe eines Blutultrafiltrats, das möglicherweise in einer Salzlösung verdünnt ist, absorbiert wird.

## Claims

1. Device for detecting a conduit (2) and for determining at least one characteristic of its content, comprising:
- a seat (1) for housing at least one portion of the conduit (2), comprising a first and a second zone (3, 4) which are opposite each other with respect to a recess intended to receive the conduit (2);
- light emission means (6), arranged in the first zone (3), having a preferential emission direction oriented towards the recess and reception means (7), sensitive to the light emitted by the emission means (6), having a preferential reception direction oriented towards the recess; and
- first light deflection means (8) for deflecting, in the direction of the first zone (3), at least part of the light emitted by the emission means (6);
characterized in that it includes second light deflection means (9) for deflecting, in the direction of the second zone (4), at least part of the light deflected by the first deflection means (8) so that, when a conduit (2) containing a substantially transparent liquid is engaged in the seat (1), at least part of the light emitted by the emission means (6) reaches the reception means (7) after having passed through the conduit (2) at least twice obliquely with respect to a longitudinal axis (5) of the recess.

2. Device according to Claim 1, characterized in that the reception means (7) are arranged in the first zone (3) and in that the emission means (6), the reception means (7), the first deflection means (8) and the second deflection means (9) are arranged with respect to each other so that, when a conduit (2) containing a substantially transparent liquid is engaged in the seat (1), at least part of the light emitted by the emission means (6) reaches the reception means (7) after having passed through the conduit (2) four times.

3. Device according to one of Claims 1 and 2, characterized in that the emission means (6), the reception means (7), the first deflection means (8) and the second deflection means (9) are arranged with respect to each other, so that, in the absence of a conduit (2) in the seat (1), at least part of the light emitted by the emission means (6) reaches the reception means (7) after a single deflection.

4. Device according to one of Claims 1 to 3, characterized in that the emission means (6), the reception means (7), the first deflection means (8) and the second deflection means (9) are arranged with respect to each other so that, when a conduit (2) containing a gas is engaged in the seat (1), the reception means (7) receive no light coming from the emission means (6).

5. Device according to one of Claims 2 to 4, characterized in that:
- the first reflection means and the second reflection means are plane mirrors (8, 9) having their reflecting surfaces arranged in planes parallel to the longitudinal axis (5) of the recess;
- the emission means (6) are arranged in order to have a preferential emission direction making an angle of approximately 135°, alternatively 45°, with respect to the longitudinal axis (5) of the recess, and
- the reception means (7) are arranged in order to have a preferential reception direction making an angle of approximately 45°, alternatively 135°, with respect to the longitudinal axis (5) of the recess.

6. Device according to Claim 1 characterized in that the reception means (7) are arranged in the second zone (4) and in that the emission means (6), the reception means (7), the first deflection means (8) and the second deflection means (9) are arranged with respect to each other so that, when a conduit (2) containing a substantially transparent liquid is engaged in the seat (1), at least part of the light emitted by the emission means (6) reaches the reception means (7) after having passed through the conduit (2) three times.

7. Device according to one of Claims 1 to 6 for a use as blood detector in a spent-liquid line of an apparatus for treating blood by means of extracorporeal circulation, characterized in that the wavelength of the light emitted by the emission means (6) is chosen to be outside the spectrum of visible light and not to be absorbed by a liquid having the coloration of a blood ultrafiltrate possibly diluted in a saline solution.
